# EUROPÄISCHE PATENTSCHRIFT

(11) **EP 0 757 029 B1**
(45) Veröffentlichungstag und Bekanntmachung des Hinweises auf die Patenterteilung: **07.11.2001**
(21) Anmeldenummer: 96111750.4
(22) Anmeldetag: 22.07.1996
(51) Int. Cl.: C07C 68/02, C07C 69/96

(54) **Verfahren zur kontinuierlichen Herstellung von Diarylcarbonaten**
Process for the continuous preparation of diaryl carbonates
Procédé de préparation continue de carbonates de diaryle

(30) Priorität: 02.08.1995 DE 19528298
(43) Veröffentlichungstag der Anmeldung: 05.02.1997
(73) Patentinhaber: BAYER AG, 51368 Leverkusen (DE)
(72) Erfinder: Ooms, Pieter, Dr., 47800 Krefeld (DE); Buysch, Hans-Josef, Dr., 47809 Krefeld (DE); Kühling, Steffen, Dr., 40670 Meerbusch (DE); Zaby, Gottfried, Dr., 51375 Leverkusen (DE)

(56) Entgegenhaltungen:
- EP-A- 0 483 632
- EP-A- 0 635 476
- EP-A- 0 645 364

## Beschreibung

Die vorliegende Erfindung betrifft ein Verfahren zur kontinuierlichen Herstellung von Diarylcarbonaten durch Umsetzung von aromatischen Hydroxyverbindungen mit Phosgen in Gegenwart von heterogenen Katalysatoren.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifüng von Phosgen oder Chlorkohlensäureester stattfinden kann, große Mengen Kochsalz als Nebenprodukt anfallen und das Lösungsmittel zurückgewonnen werden muß.

Vorschläge für Verfahren ohne Lösungsmittel finden sich z.B. in US-A 2 837 555; 3 234 263; 2 362 865. Es werden jedoch lösliche Katalysatoren verwendet, deren Abtrennung von den Produkten aufwendig ist.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird in der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. Weiter werden in der WO 91/06526 Metallsalze auf porösen Trägern als Katalysatoren für die erfindungsgemäßen Umsetzungen beschrieben. Eine vollkontinuierliche Phosgenierung von Phenol an solchen Katalysatoren ist nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Bisher gibt es also noch keinen Lösungsvorschlag für eine kontinuierliche Verfahrensweise zur Herstellung von Diarylcarbonaten durch Phosgenierung von aromatischen Hydroxyverbindungen in flüssiger Phase in Gegenwart von heterogenen Katalysatoren.

Ein solches Verfahren wurde nun gefunden. Es ist gekennzeichnet dadurch, daß man
a) ein flüssiges Gemisch aus aromatischer Hydroxyverbindung und gegebenenfalls deren Chlorameisensäureester zusammen mit Phosgen in einen mit heterogenem Katalysator gefüllten Reaktor kontinuierlich einleitet, wobei das molare Verhältnis von aromatischer Hydroxyverbindung und Phosgen von 2:1 bis 8:1 beträgt,
b) und der Reaktor adiabatisch bei Temperaturen von 130 bis 220°C und Drücken von 0,8 bis 6 bar so betriebenen wird, dass die Reaktionswärme durch Verdampfen der Edukte und Produkte abgeführt wird und die Reaktionstemperatur um maximal 50°C über die Eintrittstemperatur des Reaktionsgemisches steigt,
c) das den Reaktor verlassende Produkt entgast und das Abgas abkühlt und gegebenenfalls einem schmelzflüssigen Strom der aromatischen Hydroxyverbindung der Formel ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden,
d) das dem Reaktor entnommene und entgaste Reaktionsprodukt einem Nachreaktor zugeführt wird, in dem restlicher Chlorameisensäureester über heterogenem Katalysator bei Temperaturen von 140 bis 240°C und bei Drücken von 0,8 bis 4 bar mit noch vorhandener oder zugespeister aromatischer Hydroxyverbindung der Formel ArOH weiter zu Diarylcarbonat umgesetzt wird,
e) das den Nachreaktor verlassende Produkt entgast wird und gegebenenfalls das Abgas abkühlt und einem schmelzflüssigen Strom der aromatischen Hydroxyverbindung ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden,
f) das entgaste Reaktionsprodukt in eine erste Destillationskolonne eingespeist wird, in der aromatische Hydroxyverbindung der Formel ArOH und gegebenenfalls noch vorhandener Chlorameisensäureester über Kopf abdestilliert und dem ersten Reaktor wieder zugeführt werden und
g) der Sumpf dieser ersten Kolonne einer zweiten Destillationskolonne zugeleitet wird, in der gegebenenfalls noch vorhandene Spuren Leichtsieder aus dem Diarylcarbonat entfernt und in den oberen Teil der ersten Kolonne zurückgeführt werden und
h) aus dem Gasraum der zweiten Kolonne reines Diarylcarbonat ausgespeist wird, und
i) der Sumpf der zweiten Kolonne einer dritten Destillationseinheit zugeführt wird, in der über Kopf Diarylcarbonat abdestilliert und in die zweite Kolonne zurückgeleitet wird und
j) aus dem Sumpf der dritten Destillationseinheit die Hochsieder entnommen werden.

Aromatische Hydroxyverbindungen für das erfindungsgemäße Verfahren sind solche der Formel ArOH, worin
- Ar: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch einen oder mehrere Substituenten wie geradkettige oder verzweigte C₁-C₄-Alkyl-, C₁-C₄-Alkenyl-, C₁-C₄-Alkoxygruppen, Phenylreste oder Nitril- und Halogenfunktionen substituiert sein können, und wobei weiterhin die heterocyclischen Reste mit einem ankondensierten Benzolkern verbunden sein können.

Beispiele für erfindungsgemäße, aromatische Hydroxyverbindungen sind: Phenol, o-, m- und p-Kresol, o-, m- und p-Isopropylphenol, die entsprechenden Halogen- bzw. Alkoxyphenole, wie p-Chlorphenol bzw. p-Methoxyphenol, ferner Monohydroxyverbindungen des Naphthalins, Anthracens und Phenanthrens, weiterhin 4-Hydroxypyridin und Hydroxychinoline. Vorzugsweise werden substituierte Phenole, ganz besonders bevorzugt Phenol selbst eingesetzt.

Geeignete Katalysatoren für das erfindungsgemäße Verfahren sind im Prinzip bekannt, beispielsweise aus EP-A 483 632, EP-A 635 476, US-A 5 478 961, EP-A 635 477, US-A 5 473 094, EP-A 645 364, EP-A 691 326, EP-A 516 355, US-A 5 239 105 und US-A 5 136 077.

Die Edukte Phosgen und Hydroxyverbindung setzt man in Molverhältnissen von 1 : 2 bis 1: 8 ein. Das stöchiometrische Verhältnis ist in diesem Fall 1 : 2.

Die Katalysatoren werden in der Regel als körniges Material, Granulate, Extrudate, Stäbchen, Kugeln, oberflächenreiche Formkörper wie Hohlextrudate in Form von Raschigringen, Hohlzylindern, Sternen, Wagenrädern oder als Bruchstücke eingesetzt. Durchmesser und Länge dieser Teilchen betragen 0,5 bis 10 mm. Sie werden im Reaktor in Form einfacher Schüttungen angeordnet.

Geeignete Reaktoren für das erfindungsgemäße Verfahren sind dem Fachmann bekannt. Beispiele sind Röhrenreaktoren, gegebenenfalls mit Kühl- bzw. Heizmantel, die den Katalysator als Schüttung enthalten, oder Hordenreaktoren, in denen der Katalysator als gleichmäßige Schicht auf mehrere übereinanderliegende Böden verteilt ist.

Phosgen und aromatische Hydroxyverbindung können zur Umsetzung im Gleich- oder im Gegenstrom durch den Reaktor geleitet werden. Bei senkrecht stehenden Reaktoren kann man die flüssige Phase sowohl von oben nach unten als auch von unten nach oben durch den Reaktor leiten.

Die Umsetzung von Phosgen und aromatischer Hydroxyverbindung wird durchgeführt bei Temperaturen von 130 bis 220°C. Die Reaktionswärme wird durch Verdampfen von Edukten und Produkten soweit abgeführt, daß die Temperatur des Reaktionsgemisches um maximal 50°C, bevorzugt höchstens 40°C, besonders bevorzugt. nicht mehr als 35°C, über die Eintrittstemperatur der Reaktanden steigt.

Der Druck bewegt sich im Bereich von 0,8 bis 6 bar.

Das bei der Reaktion gebildete Abgas wird gekühlt und in einer Gegenstromapparatur einem schmelzflüssigen Strom der Hydroxyverbindung, der auch Chlorameisensäureester in Mengen von <50 Gew.-%, vorzugsweise <30 Gew.- %, besonders bevorzugt <10 Gew.-% enthalten kann, entgegengeschickt, wobei dem Gasstrom restliches Phosgen und gegebenenfalls noch mitgeführte kleinere Mengen an Hydroxyverbindungen und Chlorameisensäureester entzogen werden. Das die Gegenstromapparatur verlassende flüssige Gemisch wird durch Zugabe von Phosgen und gegebenenfalls weiterer Hydroxyverbindung auf das gewünschte Molverhältnis eingestellt, auf die gewünschte Temperatur erhitzt und dem Reaktor zugeführt.

Die Gegenstromapparatur kann beispielsweise eine Füllkörperkolonne sein, eine Bodenkolonne mit Siebböden, eine Blasensäulenkaskade oder eine kaskadierte Blasensäule, die in einem senkrechten Rohr mehrere übereinander angeordnete und über den Gasraum und Überlaufrohre oder -wehre miteinander verbundene Blasensäulen enthält.

Der am Kopf der Gegenstromapparatur austretende Gasstrom besteht im wesentlichen aus Chlorwasserstoff. Noch eventuell vorhandene Phosgenspuren können nach bekannten Methoden in einem A-Kohleturm mit wenig Wasser hydrolysiert werden. Die entsprechend ihrem Dampfdruck bei der in der Gegenstromapparatur herrschenden Temperatur noch im Chlorwasserstoffstrom befindliche Menge der Hydroxyverbindung wird in der nachfolgenden adiabatischen Absorption des Chlorwasserstoffes in Wasser durch azeotrope Destillation als wäßrige. Mischung ausgetrieben und kann nach Wiedergewinnung dem Reaktor zugeführt oder aber für andere Zwecke, wie der Herstellung von Phenolharzen verwendet werden.

Die restlichen Phosgenmengen, die sich noch im Chlorwasserstoff befinden, können auch vorteilhaft nach der adiabatischen Absorption mit Wasser, wo sie mit dem Azeotrop aus Hydroxyverbindung und Wasser ausgetrieben werden, mit den restlichen aus den Eduktströmen stammenden Inertgasspuren dem A-Kohleturm zugeführt und darin hydrolysiert werden.

Nach der Entgasung des Reaktionsgemisches erhält man ein erstes Rohprodukt, das in der Regel überwiegend aus Diarylcarbonat und/oder aromatischer Hydroxyverbindung besteht, und das noch gewisse Mengen an Chlorameisensäureester enthält, die in der Regel <50 Gew.-%, bevorzugt <30 Gew.-%, ganz besonders bevorzugt <15 Gew.-% betragen.

Dieses Gemisch kann man direkt der destillativen Aufarbeitung zuführen und in Ströme aus Chlorameisensäureester und Hydroxyverbindung, einem Diarylcarbonat und kleinen Mengen Hochsieder aufteilen. Zur Vereinfachung und ökonomischeren Durchführung der Destillation ist jedoch ein Reaktionsgemisch besser geeignet, das keinen oder nur kleine Mengen an Chlorameisensäureester enthält.

Daher leitet man vorteilhafterweise das nach der Entgasung erhaltene erste Rohprodukt in einen zweiten, heterogenen Katalysator enthaltenden Reaktor und setzt darin den noch vorhandenen Chlorameisensäureester mit im Gemisch noch befindlicher oder zugesetzter Hydroxyverbindung unter ähnlichen Bedingungen wie im ersten Reaktor um. Dabei kann der Druck in engeren Grenzen von 0,8 bis 4 bar und die Temperatur etwas höher, nämlich von 140 bis 240°C, bevorzugt 160 bis 230°C liegen.

Die Belastung der Reaktoren, gemessen in Kilogramm Eduktgemisch pro Liter Katalysatorvolumen und Stunde hängt von der Reaktionstemperatur, der Aktivität der Katalysatoren und dem gewünschten Umsatz ab. Sie beträgt 0,01 bis 20, vorzugsweise 0,02 bis 10, besonders bevorzugt 0,05 bis 4, ganz besonders bevorzugt 0,1 bis 3 kg/l h.

Auch das den Nachreaktor verlassende Gemisch wird entgast, der Abgasstrom ebenfalls in die Gegenstromapparatur geleitet und gewaschen. Das entgaste Gemisch, das nur noch geringe Mengen (<3, bevorzugt <2, besonders bevorzugt <1 Gew.-%) Chlorameisensäureester enthält, wird in einer ersten Destillationskolonne von überschüssiger Hydroxyverbindung und Chlorameisensäureester befreit, die als Kopfprodukt abgenommen und je nach Bedarf in den ersten oder zweiten Reaktor geleitet und weiter umgesetzt werden.

Das am Boden dieser Kolonne ablaufende Gemisch wird in einer zweiten Kolonne in restliche Leichtsieder, die in den oberen Teil der ersten Kolonne zurückgeführt werden, reines Diarylcarbonat, das seitlich aus dem Dampfstrom dieser zweiten Kolonne entnommen wird, und in ein Gemisch aus Diarylcarbonat und Hochsieder, das die Kolonne als Sumpf verläßt, aufgetrennt.

Diesen Sumpf trennt man in einer dritten Destillationsvorrichtung, die kontinuierlich oder diskontinuierlich betrieben wird, in einen die Hochsieder enthaltenden Sumpf und Diarylcarbonat auf, das in den unteren Teil der zweiten Kolonne geleitet und dort weiter gereinigt wird.

Die vergleichsweise geringen Mengen Sumpf von <3, vorzugsweise <2, besonders bevorzugt <1 % des Reaktionsproduktes werden zweckmäßig verbrannt oder zur Phenolharzherstellung eingesetzt.

### Beispiel

### Kontinuierliche Herstellung von Diphenylcarbonat durch Phosgenierung von Phenol in Gegenwart von γ-Aluminiumoxid

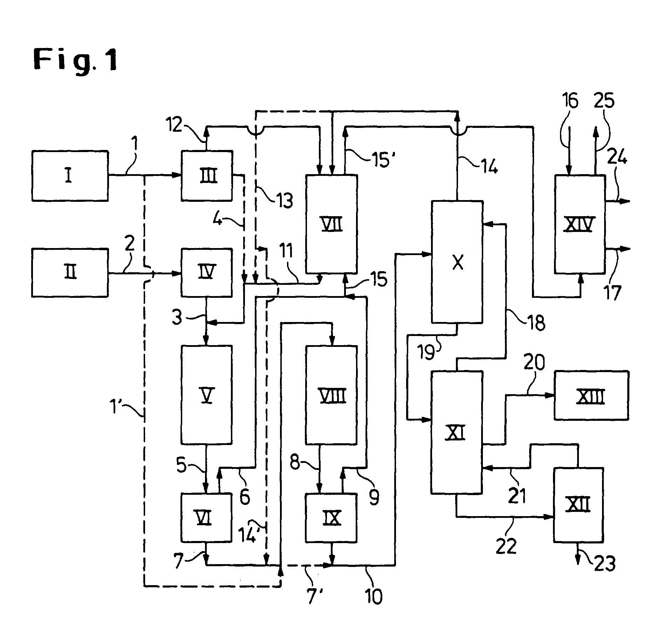

Die zur Durchführung des erfindungsgemäßen Verfahrens eingesetzte Apparatur und die auftretenden Stoffströme sind schematisch in Figur 1 wiedergegeben.

Aus einem beheizten Behälter I dosiert man über Wärmetauscher III (60°C) unter Normaldruck 41,12 Gew.-Teile/h Phenol 1 von oben in eine auf 60°C beheizte mit Füllkörpern beschickte Gegenstromkolonne VII, in welcher eine Vermischung mit aus der Destillationskolonne X am Kopf entnommenen Phenol 14 stattfindet. Nach Durchströmen des aus den Entgasungsapparaturen VI und IX stammenden Abgasstroms 15 durch VII wird Mischung 11, (Gewichtsverhältnis Phenol/Phosgen ca. 97/3) am Fuß ausgespeist. 21,93 Gew.-Teile/h über Wärmetauscher IV (170°C) vorgeheiztes Phosgen 3 wird zusammen mit 11 in einem auf 170°C beheizten, mit 150 Vol.-Teilen γ-Aluminiumoxid gefüllten Reaktor V in Gleichstrom eingeleitet.

Das am Fuß des Reaktors austretende Produkt 5, das Phenol, Chlorameisensäurephenylester, Diphenylcarbonat und Nebenprodukte im Verhältnis 56,1/0,8/ 42,8/0,3 enthält, wird über Entgaser VI in Abgas 6 (Gewichtsverhältnis Phenol, Phosgen, Chlorwasserstoff und Kohlendioxid 2,5/15,8/81,1/0,6) und Sumpf 7 (Gewichtsverhältnis Phenol, Chlorameisensäurephenylester, Diphenylcarbonat und Nebenprodukte 55,8/0,9/43,0/0,3) aufgetrennt.

Der im Sumpf 7 vorhandene Chlorameisensäurephenylester wird durch Nachreaktion mit vorhandenem Phenol (eventuell nach Zugabe von zusätzlichem Phenol (1' oder 14') in einem Nachreaktor VIII, ebenfalls befüllt mit γ-Aluminiumoxid (150 Vol.-Teilen) bei 180°C zu Diphenylcarbonat umgesetzt.

Das am Reaktorfuß entnommene Produkt 8 (Gewichtsverhältnis Phenol, Diphenylcarbonat und Nebenprodukte 55,4/44,3/0,3) wird über Entgaser IX in Abgas 9 (Phenol/Chlorwasserstoff) und Sumpf 10 (Gewichtsverhältnis Phenol, Diphenylcarbonat und Nebenprodukte 55,2/44,5/0,3) aufgetrennt.

Abgasströme 6 und 9 werden vereinigt zu 15 (Gewichtsverhältnis Phenol, Chlorwasserstoff und Kohlendioxid 5,8/93,6/0,6) und in der Gegenstromapparatur VII durch Phenol geleitet.

Das am Kopf austretende Abgas 15' wird einer Chlorwasserstoffabsorptionsanlage XIV zugeführt.

Durch Einspeisung von 94,3 Gew.-Teilen/h einer 18 %igen Salzsäure 16 erhält man 110,5 Gew.-Teile/h einer 30 %igen Salzsäure 17, die der Elektrolyse zugeführt werden kann. Das aus der Elektrolyse gewonnene Chlor kann wieder für die Herstellung von Phosgen verwendet werden.

Spuren mitgeführtes Phenol können als Azeotrop mit Wasser 24 entfernt werden.

Zur Zersetzung noch vorhandenen Phosgens im Abgas 25 wird eine Vernichtungsanlage (Aktivkohletürme mit Wasser) angeschlossen.

Sumpf 10 wird in eine erste Destillationskolonne X eingespeist und bei ca. 80°C/16 mbar (12 mm) aufgetrennt in 57,7 Gew.-Teile/h Phenol und Sumpf 19 (Gewichtsverhältnis Phenol, Diphenylcarbonat und Nebenprodukte 0,3/99,1/0,6).

Sumpf 19 wird einer zweiten Destillationskolonne XI zugeleitet, in der über Kopf noch vorhandenes Phenol (0,14 Gew.-Teile) entfernt und in den oberen Teil der ersten Kolonne X zurückgegeben wird. Das am Fuß entnommene Produkt 22 (Gewichtsverhältnis Diphenylcarbonat/Nebenprodukte 88,6/11,4) wird in einer dritten Destillationskolonne XII bei 170°C/12mm in Kopfprodukt 21 (2,3 Gew.-Teile/h Diphenylcarbonat), das in den unteren Teil der zweiten Kolonne XI zurückgeführt wird, und Sumpf 23 (hochsiedende Nebenprodukte) getrennt.

Durch seitliche Ausspeisung aus dem Gasraum der 2. Kolonne XI erhält man 46,6 Gew.-Teile/h Produkt 20 (Gewichtsverhältnis Diphenylcarbonat, Phenol 99,8/0,2).

### Beispiel 2

Durchführung des erfindungsgemäßen Verfahrens wie in Beispiel 1 beschrieben, aber ohne Gegenstromapparatur VII unter Einspeisung von 32,0 Gew.-Teilen/h Phenol 12 über Wärmetauscher III und Einleitung von 10,7 Gew.-Teilen/h über Wärmetauscher IV vorgeheiztem Phosgen 3 in Reaktor V, führt mit gleichbleibender Selektivität zu 20,1 Gew.-Teilen/h Diphenylcarbonat.

### Beispiel 3

Durchführung des erfindungsgemäßen Verfahrens wie in Beispiel 2 beschreiben, unter Einspeisung von 30,0 Gew.-Teilen/h Phenol 12 über Wärmetauscher III und Einleitung von 15,0 Gew.-Teilen/h über Wärmetauscher IV vorgeheiztem Phosgen 3 in Reaktor V, führt mit gleichbleibender Selektivität zu 24,3 Gew.-Teilen/h Diphenylcarbonat.

Als weitere Variationen zu der beschriebenen Verfahrensweise sind in Abhängigkeit von den Edukt- und Produktzusammensetzungen, Katalysatorbelastungen und Temperatur zu nennen:
a) Dosierung von Phenol 4 direkt in Reaktor V statt über Gegenstromapparatur VII beim Anfahren oder Fahrweise ohne Gegenstromapparatur.
b) Zugabe von Phenol (1' oder 14') als zusätzlicher Reaktionspartner für die Nachreaktion bei Vorliegen größerer Konzentrationen an Chlorameisensäurephenylester.
c) Fahrweise ohne Nachreaktion im Reaktor VIII, wobei dann noch vorhandener Chlorameisensäurephenylester in der ersten Destillationskolonne X als Leichtsieder 14 mit Phenol abdestilliert und in Gegenstromapparatur VII eingespeist wird, oder bei einer Fahrweise ohne Gegenstromapparatur in den ersten Reaktor V zurückgeführt wird (13).
d) Flüssige Phase und Phosgen werden im Gegenstrom durch Reaktor V geleitet. Dabei tritt Phosgen aus dem Vorratsbehälter II über Wärmetauscher IV von unten in den Reaktor V ein, die flüssige Phase von oben. Zur Abführung des gebildeten Abgases wird der obere Teil des Reaktors V zusätzlich mit Gegenstromkolonne VII verbunden.
e) Entfernung des noch im Abgasstrom 15' befindlichen Phenols durch Ausfrieren in einer Kühlfalle und Rückführung in Gegenstromapparatur VII, Reaktor V oder Nachreaktor VIII.
f) Reinigung des Diarylcarbonats 20 in einer vierten Destillationseinheit, wobei reines Diarylcarbonat als Kopfprodukt abdestilliert und Restmengen Hochsieder als Sumpfprodukt zusammen mit Sumpf 22 aus der zweiten Kolonne der dritten Destillationseinheit zugeführt werden.

## Patentansprüche

1. Verfahren zur kontinuierlichen Herstellung von Diarylcarbonaten durch Umsetzung von Phosgen mit aromatischen Hydroxyverbindungen der Formel ArOH in Gegenwart heterogener Katalysatoren, **dadurch gekennzeichnet, dass** man
a) ein flüssiges Gemisch aus aromatischer Hydroxyverbindung und gegebenenfalls deren Chlorameisensäureester zusammen mit Phosgen in einen mit heterogenem Katalysator gefüllten Reaktor kontinuierlich einleitet, wobei das molare Verhältnis von aromatischer Hydroxyverbindung und Phosgen von 2:1 bis 8:1 beträgt,
b) und der Reaktor adiabatisch bei Temperaturen von 130 bis 220°C und Drücken von 0,8 bis 6 bar so betrieben wird, dass die Reaktionswärme durch Verdampfen der Edukte und Produkte abgeführt wird und die Reaktionstemperatur um maximal 50°C über die Eintrittstemperatur des Reaktionsgemisches steigt,
c) das den Reaktor verlassende Produkt entgast, das Abgas abkühlt und einem schmelzflüssigen Strom der aromatischen Hydroxyverbindung der Formel ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden,
d) das dem Reaktor entnommene und entgaste Reaktionsprodukt einem Nachreaktor zugeführt wird, in dem restlicher Chlorameisensäureester über heterogenem Katalysator bei Temperaturen von 140 bis 240°C und bei Drücken von 0,8 bis 4 bar mit noch vorhandener oder zugespeister aromatischer Hydroxyverbindung der Formel ArOH weiter zu Diarylcarbonat umgesetzt wird,
e) das den Nachreaktor verlassende Produkt entgast wird, das Abgas abkühlt und einem schmelzflüssigen Strom der aromatischen Hydroxyverbindung ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden,
f) das entgaste Reaktionsprodukt in eine erste Destillationskolonne eingespeist wird, in der aromatische Hydroxyverbindung der Formel ArOH und gegebenenenfalls noch vorhandener Chlorameisensäureester über Kopf abdestilliert und dem ersten Reaktor wieder zugeführt werden und
g) der Sumpf dieser ersten Kolonne einer zweiten Destillationskolonne zugeleitet wird, in der gegebenenfalls noch vorhandene Spuren Leichtsieder aus dem Diarylcarbonat entfernt und in den oberen Teil der ersten Kolonne zurückgeführt werden und
h) aus dem Gasraum der zweiten Kolonne reines Diarylcarbonat ausgespeist wird, und
i) der Sumpf der zweiten Kolonne einer dritten Destillationseinheit zugeführt wird, in der über Kopf Diarylcarbonat abdestilliert und in die zweite Kolonne zurückgeleitet wird und
j) aus dem Sumpf der dritten Destillationseinheit die Hochsieder entnommen werden.

2. Verfahren gemäß Anspruch 1, bei dem das den Reaktor verlassende Produkt entgast wird und das Abgas abgekühlt und einem schmelzflüssigen Strom der aromatischen Hydroxyverbindung ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden.

3. Verfahren gemäß Anspruch 2, bei dem das dem Reaktor entnommene und entgaste Reaktionsprodukt einem Nachreaktor zugeführt wird, in dem restlicher Chlorameisensäureester über heterogenem Katalysator bei Temperaturen von 140 bis 240°C und bei Drücken von 0,8 bis 4 bar mit noch vorhandener oder zugespeister aromatischer Hydroxyverbindung der Formel ArOH weiter zu Diarylcarbonat umgesetzt wird.

4. Verfahren gemäß Anspruch 3, bei dem das den Nachreaktor verlassende Produkt entgast wird und das Abgas abgekühlt und einem schmelzflüssigen Strom der aromatischen Hydroxyverbindung ArOH, der gegebenenfalls etwas Chlorameisensäureester enthält, entgegengeschickt wird, wobei Phosgen, aromatische Hydroxyverbindung und deren Chlorameisensäureester aus dem Abgasstrom entfernt werden.

5. Verfahren gemäß Anspruch 2 oder 4, bei dem a) das entgaste Reaktionsprodukt in eine erste Destillationskolonne eingespeist wird, in der aromatische Hydroxyverbindung ArOH und gegebenenfalls noch vorhandener Chlorameisensäureester über Kopf abdestilliert und dem ersten Reaktor wieder zugeführt werden, b) der Sumpf dieser ersten Kolonne einer zweiten Destillationskolonne zugeleitet wird, in der gegebenenfalls noch vorhandene Spuren Leichtsieder aus dem Diarylcarbonat entfernt und in den oberen Teil der ersten Kolonne zurückgeführt werden, c) aus dem Gasraum der zweiten Kolonne reines Diarylcarbonat ausgespeist wird, d) der Sumpf der zweiten Kolonne einer dritten Destillationseinheit zugeführt wird, in der über Kopf Diarylcarbonat abdestilliert und in die zweite Kolonne zurückgeleitet wird, und e) aus dem Sumpf der dritten Destillationseinheit die Hochsieder entnommen werden.

## Claims

1. Process for the continuous production of diaryl carbonates by reaction of phosgene with aromatic hydroxy compounds of the formula ArOH in the presence of heterogeneous catalysts, **characterised in that**
a) a liquid mixture consisting of aromatic hydroxy compound and optionally the chloroformates thereof is continuously introduced together with phosgene into a reactor that is filled with heterogeneous catalyst, the molar ratio of aromatic hydroxy compound to phosgene amounting to from 2:1 to 8:1,
b) and the reactor is operated adiabatically at temperatures from 130 to 220°C and at pressures from 0.8 to 6 bar in such a way that the heat of reaction is dissipated by evaporation of the educts and products and the reaction temperature rises by a maximum of 50°C above the admission temperature of the reaction mixture,
c) the product leaving the reactor is degassed, the waste gas is cooled and sent towards a molten flow of the aromatic hydroxy compound of the formula ArOH, which optionally contains some chloroformate, whereby phosgene, aromatic hydroxy compound and the chloroformate thereof are removed from the stream of waste gas,
d) the reaction product that has been withdrawn from the reactor and degassed is supplied to a secondary reactor in which residual chloroformate is caused to react further, over heterogeneous catalyst at temperatures from 140 to 240°C and at pressures from 0.8 to 4 bar, with aromatic hydroxy compound of the formula ArOH that is still present or that has been fed in, so as to form diaryl carbonate,
e) the product leaving the secondary reactor is degassed, the waste gas is cooled and sent towards a molten flow of the aromatic hydroxy compound ArOH, which optionally contains some chloroformate, whereby phosgene, aromatic hydroxy compound and the chloroformate thereof are removed from the stream of waste gas,
f) the degassed reaction product is fed into a first distillation column in which aromatic hydroxy compound of the formula ArOH and chloroformate which is optionally still present are distilled off overhead and resupplied to the first reactor and
g) the sump of this first column is conducted to a second distillation column in which traces of low-boiling components which are optionally still present are removed from the diaryl carbonate and recycled into the upper part of the first column and
h) pure diaryl carbonate is discharged from the gas space of the second column, and
i) the sump of the second column is supplied to a third distillation unit in which diaryl carbonate is distilled off overhead and conducted back into the second column and
j) the high-boiling components are withdrawn from the sump of the third distillation unit.

2. Process according to Claim 1, wherein the product leaving the reactor is degassed and the waste gas is cooled and sent towards a molten flow of the aromatic hydroxy compound ArOH, which optionally contains some chloroformate, whereby phosgene, aromatic hydroxy compound and the chloroformate thereof are removed from the stream of waste gas.

3. Process according to Claim 2, wherein the reaction product that has been withdrawn from the reactor and degassed is supplied to a secondary reactor in which residual chloroformate is caused to react further, over heterogeneous catalyst at temperatures from 140 to 240°C and at pressures from 0.8 to 4 bar, with aromatic hydroxy compound of the formula ArOH that is still present or that has been fed in, so as to form diaryl carbonate.

4. Process according to Claim 3, wherein the product leaving the secondary reactor is degassed and the waste gas is cooled and sent towards a molten flow of the aromatic hydroxy compound ArOH, which optionally contains some chloroformate, whereby phosgene, aromatic hydroxy compound and the chloroformate thereof are removed from the stream of waste gas.

5. Process according to Claim 2 or 4, wherein a) the degassed reaction product is fed into a first distillation column in which aromatic hydroxy compound ArOH and chloroformate which is optionally still present are distilled off overhead and resupplied to the first reactor, b) the sump of this first column is conducted to a second distillation column in which traces of low-boiling components which are optionally still present are removed from the diaryl carbonate and recycled into the upper part of the first column, c) pure diaryl carbonate is discharged from the gas space of the second column, d) the sump of the second column is supplied to a third distillation unit in which diaryl carbonate is distilled off overhead and conducted back into the second column, and e) the high-boiling components are withdrawn from the sump of the third distillation unit.

## Revendications

1. Procédé de préparation continue de carbonates de diaryle par réaction de phosgène avec un composé hydroxylé aromatique de formule ArOH, en présence d'un catalyseur hétérogène, **caractérisé en ce que** :
a) on introduit en continu, un mélange liquide du composé hydroxylé aromatique et le cas échéant, de son ester d'acide chloroformique avec du phosgène dans un réacteur rempli d'un catalyseur hétérogène, où le rapport molaire du composé hydroxylé aromatique au phosgène se situe dans l'intervalle allant de 2:1 à 8:1, et
b) on fait fonctionner le réacteur de manière adiabatique à des températures allant de 130 à 220°C et des pressions allant de 0,8 à 6 bar, de sorte que la chaleur de réaction est éliminée par évaporation des réactifs et produits et la température de réaction s'élève à au maximum 50°C au-dessus de la température d'entrée du mélange réactionnel,
c) on dégaze le produit quittant le réacteur, le gaz résiduaire est refroidi et est envoyé à contre-courant du courant fondu du composé hydroxylé aromatique de formule ArOH, qui contient, le cas échéant, un peu d'ester d'acide chloroformique, où le phosgène, le composé hydroxylé aromatique et son ester d'acide chloroformique sont éliminés du courant de gaz résiduaire,
d) le produit de réaction prélevé du réacteur et dégazé est conduit dans un réacteur ultérieur, dans lequel l'ester d'acide chloroformique résiduel est mis à réagir sur le catalyseur hétérogène à des températures allant de 140 à 240°C et à des pressions allant de 0,8 à 4 bar, avec le composé hydroxylé aromatique de formule ArOH, encore présent ou ajouté, en carbonate de diaryle,
e) on dégaze le produit quittant le réacteur ultérieur, le gaz résiduaire est refroidi et est envoyé à contre-courant du courant fondu du composé hydroxylé aromatique ArOH, qui contient, le cas échéant, un peu d'ester d'acide chloroformique, où le phosgène, le composé hydroxylé aromatique et son ester d'acide chloroformique sont éliminés du courant de gaz résiduaire,
f) on introduit le produit de réaction dégazé dans une première colonne de distillation, dans laquelle le composé hydroxylé aromatique de formule ArOH et l'ester d'acide chloroformique, le cas échéant, encore présent sont distillés par la tête et sont ramenés dans le premier réacteur, et
g) le bas de cette première colonne est conduit vers une deuxième colonne de distillation, dans laquelle les traces, le cas échéant encore présentes, de produits de bas point d'ébullition sont séparées du carbonate de diaryle et ramenées dans la partie supérieure de la première colonne,
h) on prélève le carbonate de diaryle pur dans le chambre de gaz de la deuxième colonne,
i) le bas de la deuxième colonne est amené dans une troisième unité de distillation, dans laquelle le carbonate de diaryle distille par la tête et est ramené dans la deuxième colonne, et
j) on prélève dans le bas de la troisième unité de distillation, les produit de haut point d'ébullition.

2. Procédé suivant la revendication 1, dans lequel le produit quittant le réacteur est dégazé, le gaz résiduaire est refroidi et est envoyé à contre-courant du courant fondu du composé hydroxylé aromatique ArOH, qui contient, le cas échéant, un peu d'ester d'acide chloroformique, où le phosgène, le composé hydroxylé aromatique et son ester d'acide chloroformique sont éliminés du courant de gaz résiduaire.

3. Procédé suivant la revendication 2, dans lequel le produit de réaction prélevé du réacteur et dégazé est conduit vers un réacteur ultérieur, dans lequel l'ester d'acide chloroformique résiduel est mis à réagir avec le composé hydroxylé aromatique de formule ArOH encore présent ou ajouté, en carbonate de diaryle, sur le catalyseur hétérogène, à une température située dans l'intervalle allant de 140 à 240°C et une pression allant de 0,8 à 4 bar.

4. Procédé suivant la revendication 3, dans lequel le produit quittant le réacteur ultérieur est dégazé, le gaz résiduaire refroidi et envoyé à contre-courant du courant fondu du composé hydroxylé aromatique ArOH, qui contient, le cas échéant, un peu d'ester d'acide chloroformique, où le phosgène, le composé hydroxylé aromatique et son ester d'acide chloroformique sont éliminés du courant de gaz résiduaire.

5. Procédé suivant la revendication 2 ou 4, dans lequel a) le produit de réaction dégazé est alimenté dans une première colonne de distillation, dans laquelle le composé hydroxylé aromatique ArOH et l'ester d'acide chloroformique encore présent, le cas échéant, sont distillés par la tête et ramenés vers le premier réacteur, b) le fond de cette première colonne est amené dans une deuxième colonne de distillation, dans laquelle les traces, le cas échéant encore présentes, de composés à bas point d'ébullition sont séparées du carbonate de diaryle et recyclées dans la partie supérieure de la première colonne, c) le carbonate de diaryle pur est prélevé dans la chambre à gaz de la deuxième colonne, d) le fond de la deuxième colonne est amené dans une troisième unité de distillation, dans laquelle le carbonate de diaryle distille par la tête et est recyclé dans la deuxième colonne, et e) au fond de la troisième unité de distillation, on prélève les composés de haut point d'ébullition.
